# FASCICULE DE BREVET EUROPEEN

(11) **EP 3 389 731 B1**
(45) Date de publication et mention de la délivrance du brevet: **29.07.2020**
(21) Numéro de dépôt: 16826173.3
(22) Date de dépôt: 19.12.2016
(51) Int. Cl.: A01P 1/00, A01N 43/08, A01N 43/16, A01N 43/90, A61L 2/16, A61K 31/7004

(54) **COMPOSITION ANTIBACTÉRIENNE CONTENANT UN MONOÉTHER OU UN MONOACÉTAL D'ALKYLE DE DÉSOXYHEXOSE**
ANTIBAKTERIELLE ZUSAMMENSETZUNG MIT DESOXYHEXOSEALKYLMONOACETAL ODER MONOETHER
ANTIBACTERIAL COMPOSITION CONTAINING A DEOXYHEXOSE ALKYL MONOACETAL OR MONOETHER

(30) Priorité: 17.12.2015 FR 1502629
(43) Date de publication de la demande: 24.10.2018
(73) Titulaire: Tereos Starch & Sweeteners Belgium, 9300 Aalst (BE); Université Claude Bernard Lyon 1, 69622 Villeurbanne, Cedex (FR); Centre National de la Recherche Scientifique, 75016 Paris (FR)
(72) Inventeur: GOZLAN, Charlotte, 93190 Livry Gargan (FR); BELMESSIERI, Dorine, 69622 Villeurbanne Cedex (FR); DUCLOS, Marie-Christine, 69622 Villeurbanne Cedex (FR); DUGUET, Nicolas, 69622 Villeurbanne Cedex (FR); LEMAIRE, Marc, 69622 Villeurbanne Cedex (FR); LINA, Gérard, 69622 Villeurbanne Cedex (FR); DUMITRESCU, Oana, 69622 Villeurbanne Cedex (FR); REDL, Andreas, 9300 Aalst (BE)
(74) Mandataire: Icosa
(86) Numéro de dépôt international: PCT/IB2016/057780
(87) Numéro de publication internationale: WO 2017/103904

(56) Documents cités:
- SMITH A ET AL: "Synthesis and antimicrobial evaluation of carbohydrate and polyhydroxylated non-carbohydrate fatty acid ester and ether derivatives", CARBOHYDRATE RESEARCH, PERGAMON, GB, vol. 343, no. 15, 13 octobre 2008 (2008-10-13), pages 2557-2566, XP025408848, ISSN: 0008-6215, DOI: 10.1016/J.CARRES.2008.07.012 [extrait le 2008-07-26]
- FANTON E ET AL: "Long-chain acetals derived from sucrose as a new class of surfactants", CARBOHYDRATE RESEARCH, PERGAMON, GB, vol. 298, no. 1-2, 20 février 1997 (1997-02-20), pages 85-92, XP004109759, ISSN: 0008-6215, DOI: 10.1016/S0008-6215(96)00300-X
- SHUICHI MATSUMURA ET AL: "Surface activities, biodegradability and antimicrobial properties of n-alkyl glucosides, mannosides and galactosides", JOURNAL OF THE AMERICAN OIL CHEMISTS' SOCIETY, vol. 67, no. 12, 1 décembre 1990 (1990-12-01), pages 996-1001, XP055029089, ISSN: 0003-021X, DOI: 10.1007/BF02541865

## Description

### Domaine technique

La présente invention concerne une composition bactéricide ou bactériostatique comprenant un acétal d'alkyle ou un éther d'alkyle de désoxyhexose ou d'un dérivé de désoxyhexose dans lequel le groupe alkyle comprend entre 11 à 18 atomes de carbone, un sel pharmaceutiquement acceptable, un isomère ou un mélange d'isomères de celui-ci, son utilisation dans le traitement ou la prévention des infections à bactéries Gram positif, son utilisation en tant que produit d'hygiène ou dermatologique à usage externe ainsi qu'une méthode de désinfection de surfaces.

### Arrière-plan technique

Les composés antimicrobiens se définissent comme des molécules capables d'inhiber ou de stopper la croissance de micro-organismes ou de les tuer. Dans ce contexte, ils sont couramment utilisés pour prévenir ou traiter les infections humaines et animales, et dans l'industrie agroalimentaire pour prévenir la multiplication de bactéries pathogènes dans les aliments. La large gamme d'utilisation des composés antimicrobiens a favorisé l'émergence d'agents infectieux résistants. La diffusion de bactéries ayant acquis des mécanismes de résistance vis-à-vis des composés antimicrobiens les plus largement utilisés sont un problème de santé publique majeur de plus en plus alarmant (J. S. Bradley et al. Lancet Infect. Dis. 2007;7:68-78).

À titre illustratif, de nombreuses souches résistantes aux antibiotiques de l'espèce la plus pathogène du genre Staphylococcus, à savoir *Staphylococcus aureus* ont été isolées. Or, les infections à staphylocoques représentent un pourcentage important des infections graves. De plus, près de la moitié des infections nosocomiales seraient liées à un staphylocoque. On peut citer également les nombreuses souches d'*Enterococcus faecalis* ou d'*Enterococcus faecium* résistantes aux agents antibiotiques communément utilisés. Or, bien qu'elles ne soient moins virulentes par rapport aux staphylocoques notamment, on recense un nombre croissant de souches d'entérocoques multirésistantes et plus récemment des épidémies d'entérocoques résistant aux glycopeptides, les antibiotiques de recourt vis-à-vis de cette famille bactérienne.

Un autre phénomène d'antibiorésistance a été décrit qui pourrait ne pas être seulement lié à l'usage excessif d'antibiotiques, mais aux méthodes de conservation des aliments. Ainsi, par exemple il a été montré que *Listeria monocytogenes* s'avère plus résistante aux antibiotiques après avoir survécu à un stress osmotique, à une basse température ou à un milieu acide (Anas A. et al. (2015) Food Microbiology, Volume 46, April, Pages 154-160). Or, la contamination humaine est d'origine alimentaire. En outre, bien qu'elle soit relativement rare, la listériose humaine est une infection grave avec une mortalité estimée à 50 %. Ainsi, l'émergence de résistance aux antibiotiques chez *L. monocytogenes* qui pourrait être induite par les méthodes modernes de conservation ou de traitement des aliments constitue une grave menace pour la santé publique.

Bien que plusieurs mécanismes soient souvent impliqués simultanément dans la résistance aux antibiotiques, il est commun de les classer en trois catégories: (a) défaut de pénétration de l'antibiotique dans la bactérie, (b) inactivation ou excrétion de l'antibiotique par des systèmes enzymatiques bactériens et (c) défaut d'affinité entre cible bactérienne et antibiotique. Ces trois catégories de mécanisme de résistance ont une composante structurelle à savoir les mécanismes mis en œuvre sont dépendants de la structure de la molécule concernée.

Ainsi, afin d'obtenir une composition antibiotique ayant des chances réduites de permettre le développement d'une résistance, les inventeurs ont envisagé l'utilisation d'une composition contenant un mélange de composés ayant une activité antibiotique mais comportant des différences structurelles mineures susceptibles de réduire les chances de développement d'une résistance bactérienne. Ils ont ainsi envisagé une composition comprenant un mélange isomérique de composés ayant une activité antibiotique.

Les inventeurs ont souhaité développer une composition antibiotique ayant également une faible toxicité et un faible impact environnemental. Une composition antibiotique biodégradable susceptible d'être obtenue en grandes quantités à partir de ressources renouvelables, à faible coût afin d'être parfaitement accessibles pour une application industrielle mais également aussi efficaces que des antimicrobiens non biosourcés.

Or, aucun procédé de l'art antérieur ne permet d'obtenir un mélange isomérique de composés biosourcés à faible toxicité et à faible coût.

Néanmoins, des composés biosourcés ont été décrits par l'art antérieur. Ainsi, l'art antérieur décrit différents composés utilisés comme antimicrobiens parmi lesquels les acides gras et leurs correspondants d'esters polyhydroxylés actifs contre les bactéries Gram positif et possédant de longues chaînes aliphatiques. A titre indicatif, un des antimicrobiens les plus actifs est la monolaurine, un monoester de glycérol présentant une chaîne aliphatique en C12. Sa dénomination commerciale est la LAURICIDIN®. Ce composé est utilisé en tant qu'additif alimentaire dans le but d'inhiber la croissance bactérienne (E. Freese, C. W. Sheu, E. Galliers. Nature 1973, 241, 321-325*;* E. G. A. Verhaegh, D. L. Marshall, D.-H. Oh. Int. J. Food Microbiol.1996, 29, 403-410). Or, la fonction ester de la monolaurine étant sensible aux estérases, ce composé est rapidement dégradé et possède un temps de demi vie faible. Par ailleurs, Smith *et al.* décrivent la synthèse et les effets antimicrobiens d'esters et d'éthers en C8-C12 d'hexoses alkylés, notamment des esters du méthyle de glucose et de galactose(SMITH A. et al. "Synthesis and antimicrobial evaluation of carbohydrate and polyhydroxylated non-carbohydrate fatty acid ester and ether derivates" CARBOHYDRATE RESEARCH, PERGAMON, GB vol. 343, no.15, 2008, pp.2557-2566). De plus, Matsumura *et al.* décrivent la synthèse et les propriétés antimicrobiennes d'éthers en C8-C12 d'hexoses tels que le glucose, le galactose et le mannose (SHUICHI MATSUMURA et al. Journal of the American Oil Chemistry (1990-12-01) vol. 67, no. 12, pages 996-1001).

L'art antérieur décrit également des antimicrobiens dérivés de sucre considérés comme particulièrement attractifs du fait de leur biodégradabilité, leurs faibles toxicités et impact environnemental.

Des exemples d'antimicrobiens dérivés de sucre sont les esters dérivés de sucre qui sont également utilisés industriellement pour des applications antimicrobiennes car leurs matières premières et leur coût de production restent relativement peu élevés. On peut citer par exemple, le caprylate de sorbitane décrit dans la demande internationale de brevet WO2014/025413 en mélange avec de l'Hinokitiol dans une formulation antimicrobienne. Selon cette demande, cette formulation permettrait d'inhiber ou de tuer des bactéries à Gram positif et négatif, des champignons et/ou des mycoses.

L'art antérieur décrit également l'utilisation d'esters de disaccharide en tant qu'agent antimicrobien dans l'industrie alimentaire. Le dodécanoyl de sucrose est l'un des plus utilisés. Ce dernier serait particulièrement actif contre la *L. monocytogenes* (M. Ferrer, J. Soliveri, F.J. Plou, N. López-Cortés, D. Reyes-Duarte, M. Christensen, J.L. Copa-Patiño, A. Ballesteros, Enz. Microb. Tech., 2005, 36, 391-398). Néanmoins, il est également décrit comme faiblement inhibiteur de la croissance de *S. aureus*, pour des applications dans le domaine hospitalier *(*J.D. Monk, L.R. Beuchat, A.K. Hathcox, J. Appl. Microbiol., 1996, 81, 7-18*).* Ainsi, l'ester de sucrose présenterait des propriétés bactériostatiques (arrête la croissance bactérienne) mais pas bactéricides (tue les bactéries).

En outre, la synthèse d'esters de sucre présente de nombreux inconvénients. Tout d'abord, malgré le faible coût de production, la synthèse d'esters, plus particulièrement pour les di- et trisaccharides, est problématique à cause de la haute fonctionnalité des sucres qui entraîne la formation d'un mélange de mono-, di- et polyesters et la présence de solvant polaire, comme le diméthylformamide (DMF) et la pyridine, est généralement nécessaire afin de mieux solubiliser les réactifs hautement polaires. Cependant, ces solvants sont classés Cancérigène, Mutagène et Reprotoxique (CMR) et leur usage doit être évité. Pour résoudre ce problème, la synthèse enzymatique a été utilisée mais la nécessité de se placer en milieu très dilué dans ces conditions, rend la production limitée.

Par ailleurs, les fonctions esters de ces composés sont facilement hydrolysables par les estérases présentes dans les cellules. Or, les molécules libérées suite à cette hydrolyse à savoir, le sucre et l'acide gras, ne présentent pas ou peu de propriétés antimicrobiennes (l'acide gras étant légèrement actif). Ceci induit une instabilité responsable d'une réduction du temps d'activité de ces composés.

Ainsi, afin d'obtenir une composition antibiotique peu propice au développement d'une résistance comprenant des agents antimicrobiens efficaces et stables, l'invention propose un monoéther ou monoacétal d'alkyle de désoxyhexose dans lequel le groupe alkyle comprend entre 11 à 18 atomes de carbone sous forme de mélange de régioisomères et/ou les diastéréoisomères obtenu dans des conditions peu onéreuses tout en étant respectueuses pour l'environnement et ne représentant pas de danger pour des applications topiques ou par ingestion.

### Description détaillée de l'invention

### Composition bactéricide ou bactériostatique

L'invention concerne une composition typiquement, bactéricide ou bactériostatique caractérisée en ce qu'elle comprend un acétal d'alkyle ou un éther d'alkyle de désoxyhexose et/ou un acétal d'alkyle ou un éther d'alkyle de désoxyhexose glycosylé et/ou hydrogéné et/ou déshydraté dans lequel le groupe alkyle comprend entre 11 à 18 atomes de carbone, un sel pharmaceutiquement acceptable, un isomère ou un mélange d'isomères de celui-ci, ledit radical acétal alkyle ou éther alkyle est en position 2-*O*, 3-*O*-, 4-*O*-, les isomères étant choisis parmi les régioisomères et/ou les diastéréoisomères. Typiquement, le désoxyhexose est choisi parmi le rhamnose ou le fucose. Avantageusement, l'acétal d'alkyle ou l'éther d'alkyle de désoxyhexose est un monoéther ou monoacétal d'alkyle de désoxyhexose. Typiquement, ledit désoxyhexose étant un désoxyhexose glycosylé et/ou hydrogéné et/ou déshydraté.

L'invention concerne également une composition comprenant un monoéther ou monoacétal d'alkyle de désoxyhexose ou de dérivé de désoxyhexose ou un mélange d'isomères de celui-ci, ledit dérivé de désoxyhexose étant un désoxyhexose glycosylé et/ou hydrogéné et/ou déshydraté, les isomères étant choisis parmi les régioisomères et/ou les diastéréoisomères, ledit monoéther ou monoacétal d'alkyle de désoxyhexose ou de dérivé de désoxyhexose ou le mélange d'isomères de celui-ci étant obtenu par un procédé comprenant les étapes suivantes :
a) une acétalisation ou trans-acétalisation d'un désoxyhexose ou d'un dérivé de désoxyhexose par un aldéhyde aliphatique contenant de 11 à 18 atomes de carbone ou l'acétal de celui-ci
b) optionnellement, hydrogénolyse catalytique de l'acétal alkyle de désoxyhexose ou du dérivé de désoxyhexose obtenu en a) préférentiellement, sans catalyseur acide, et
c) récupération d'un mélange d'isomères de monoéthers d'alkylique de désoxyhexose ou du dérivé de désoxyhexose obtenue en b) dans lequel le groupe alkyle (R) comprend entre 11 à 18 atomes de carbone ou
   récupération d'un mélange d'isomères de monoacétals d'alkyle de désoxyhexose ou de dérivé de désoxyhexose obtenue en a) dans lequel le groupe alkyle (R) comprend entre 11 à 18 atomes de carbone.

Un « désoxyhexose » doit être compris comme étant un hexose dont un des groupements hydroxyles (OH) a été remplacé par un hydrogène. Les désoxyhexose peuvent être isolés à partir de végétaux par exemple le Rhamnose est issu du Nerprun (Rhamnus) ou du sumac, le fucose de polysaccharides membranaires de cellules de mammifères ou d'insectes. Un exemple de désoxyhexose approprié peut être, le fucose ou le rhamnose.

Tel qu'utilisé ici, le terme «rhamnose» se réfère au D-(-)-Rhamnose ou au L-(+)-Rhamnose. Egalement appelé Isodulcitol ou 6-deoxy-L-mannose, le rhamnose est un hexose de formule brute C₆H₁₂O₅.

Tel qu'utilisé ici, le terme «fucose» se réfère au D-(-)-fucose ou au L-(+)-fucose. Egalement appelé 6-deoxy-L-galactose, le fucose est un hexose de formule brute C₆H₁₂O₅.

Selon un mode de réalisation, le désoxyhexose est un désoxyhexose glycosylé et/ou hydrogéné et/ou déshydraté. De tels désoxyhexoses glycosylés et/ou hydrogénés et/ou déshydratés sont qualifiés dans la présente de « dérivés de désoxyhexose ». Par exemple, le dérivé de rhamnose est un anhydrorhamnose ou du rhamnitol.

Un « anhydrorhamnose » doit être compris comme étant un composé obtenu par déshydratation, par l'élimination d'une ou plusieurs molécules d'eau à partir du rhamnose . Un exemple d'un anhydrorhamnose peut être le 1,2-anhydrorhamnose, le 1,2-Anhydrorhamnose, le 1,3-Anhydrorhamnose, le 2,3-Anhydrorhamnose.

L'anhydrorhamnose peut être obtenu par la déshydratation du rhamnitol pour former par exemple, le 1,5-Anhydrorhamnitol.

Selon un mode de réalisation, ledit dérivé de rhamnose est un sucre-alcool, le rhamnitol. Tel qu'il est utilisé ici, le terme « sucre-alcool », également connu sous le nom «polyol» se réfère à une forme hydrogénée de monosaccharide dont le groupe carbonyle (aldéhyde ou cétone) a été réduit en un hydroxyle primaire ou secondaire.

De même, lorsque le désoxyhexose est un fucose, ledit dérivé hydrogéné du fucose est un sucre-alcool, le fucositol.

Avantageusement, le dérivé de fucose est un anhydrofucose. Un « anhydrofucose » doit être compris comme étant un fucose obtenu par déshydratation, par l'élimination d'une ou plusieurs molécules d'eau à partir du fucose . Un exemple d'un anhydrofucose peut être le 1,2-Anhydrofucose. 1,3-Anhydrofucose.

Selon un mode de réalisation, le procédé selon l'invention peut comprendre une étape de déshydratation dudit désoxyhexose ou de son dérivé afin d'obtenir par exemple un monoanhydrorhamnose ou un monoanhydrofucose. Typiquement, le désoxyhexose est fondu avant l'étape de déshydratation. L'étape de déshydratation peut être effectuée avec un catalyseur par exemple, avec un catalyseur acide.

Selon l'invention, l'étape de déshydratation est effectuée sous atmosphère d'hydrogène à une pression de préférence d'environ de 20 à 50 bar.

Avantageusement, l'étape de déshydratation est effectuée à une température comprise entre 120 et 170°C, de préférence entre 130 et 140°C.

Typiquement, le dérivé de désoxyhexose est purifié après l'étape de déshydratation, par exemple par cristallisation, recristallisation ou chromatographie.

Selon un mode de réalisation, ledit dérivé désoxyhexose est un désoxyhexose glycosylé autrement dit un alkylglycoside.

Tel qu'utilisé ici, le terme « glycosylé » ou « glycosylation » se réfère à une réaction entre un groupe alkyl et un saccharide au niveau de la position anomérique (fonction hemiacétal) du saccharide pour conduire à une fonction acétal mixte (IUPAC Compendium of Chemical Terminology Gold book Version 2.3.3 2014-02-24 p. 635-636 *et* PAC, 1995, 67, 1307 *(«* Glossary of class names of organic compounds and reactivity intermediates based on structure » IUPAC Recommendations 1995) page 1338 *White Book, p. 136).* Cette réaction de glycosylation s'oppose à l'alkylation en ce que cette dernière peut avoir lieu entre un groupe alkyl et un saccharide mais sur n'importe quel oxygène du saccharide pour former une fonction éther.

Tel qu'utilisé ici, le terme "alkylglycoside" se réfère un désoxyhexose où la partie réductrice est reliée par une liaison à un groupe alkyl par glycosylation, tel que décrit dans l'état de la technique. Typiquement, le désoxyhexose ou son dérivé peut être lié au groupement alkyle par un atome d'oxygène (un *O*-glycoside), un atome d'azote (une glycosylamine), un atome de soufre (un thioglycoside), ou un atome de carbone (un *C-*glycoside). Le groupe alkyle peut avoir une longueur de chaîne variée de préférence, le groupe alkyle est un groupe alkyle en C1-C4. Un groupe alkyle encore plus préféré est un méthyle ou un éthyle. Typiquement, le désoxyhexose glycosilé est un rhamnose glycosilé, un rhamnitol glycosilé, un fucose glycosilé, ou un fucositol glycosilé. Des alkyles glycosides peuvent être par exemple choisis parmi un groupe constitué du rhamnoside de méthyle, du rhamnoside d'éthyle, du rhamnoside de propyle, du rhamnoside de butyle, du fucoside de méthyle, du fucoside d'éthyle, du fucoside de propyle et du fucoside de butyle.

Selon l'invention, l'étape d'acétalisation ou de trans-acétalisation comprend:
i) éventuellement, une étape de préchauffage dudit désoxyhexose ou de son dérivé, de préférence à une température comprise entre 70 et 130°C, typiquement entre 90 et 110°C,
ii) une étape d'addition de l'aldéhyde aliphatique ou d'un dérivé d'aldéhyde aliphatique désoxyhexose ou à son dérivé et
iii) une étape d'addition d'un catalyseur de préférence d'un catalyseur acide.

Typiquement, l'acétal d'un aldéhyde aliphatique peut être un acétal de di-alkyle de l'aldéhyde correspondant. Les acétals de di-méthyle et les acétals de di-éthyle sont préférés.

L'étape i) est particulièrement avantageuse en ce qu'elle peut être mise en œuvre en l'absence de solvant.

De préférence, le catalyseur acide utilisé durant l'étape d'acétalisation ou de trans-acétalisation et le cas échéant lors de l'étape de déshydratation peut être un catalyseur acide homogène ou hétérogène. Le terme «homogène», tel qu'utilisé dans l'expression « catalyseur acide homogène » se réfère à un catalyseur qui se trouve dans la même phase (solide, liquide ou gaz) ou dans le même état d'agrégat que le réactif. Inversement, le terme « hétérogène », tel qu'utilisé dans l'expression « catalyseur acide hétérogène » se réfère à un catalyseur qui se trouve dans une phase différente (solide, liquide ou gaz) que les réactifs.

Ledit catalyseur acide utilisé durant l'étape d'acétalisation ou de trans-acétalisation et le cas échéant lors de l'étape de déshydratation peut être indépendamment choisi parmi les acides solides ou liquides, organiques ou inorganiques, les acides solides étant préférés. En particulier, le catalyseur acide préféré est choisi parmi l'acide para-toluène sulfonique, l'acide méthane sulfonique, l'acide camphosulfonique (CSA) et les résines sulfoniques.

Typiquement, l'étape d'acétalisation ou de trans-acétalisation est effectuée à des températures comprises entre 70 et 130°C, typiquement entre 70 et 90°C. La température des mélanges réactionnels peut varier en fonction des réactifs et solvants utilisés. Le temps de réaction est déterminé par le degré de conversion atteint.

Selon un mode de réalisation, l'étape d'acétalisation ou de trans-acétalisation peut être effectuée par un aldéhyde aliphatique ou l'acétal de celui-ci, typiquement, un aldéhyde aliphatique linéaire ou ramifié ou l'acétal de celui-ci. L'étape d'acétalisation ou de trans-acétalisation peut être typiquement réalisée avec un aldéhyde aliphatique ou l'acétal de celui-ci ayant 11, 12, 13, 14, 15, 16, 17 ou 18 atomes de carbone, par exemple choisi parmi le undécanal, le dodécanal, le tridécanal, le tétradécanal, le pentadecanal, l'hexadécanal, l'heptadecanal, l'octodécanalet l'acétal. De préférence, l'aldéhyde aliphatique en C11-C13 ou l'acétal de celui-ci est un aldéhyde aliphatique en C12 ou l'acétal de celui-ci par exemple, un dodécanal ou l'acétal de celui-ci.

L'expression « l'acétal de celui-ci » ou « leur(s) acétal(s) », telle qu'utilisée présentement englobe le di-alkyl acétal de l'aldéhyde aliphatique en C11-C18 correspondant. Plus particulièrement, les acétals de di-méthyle ou de di-éthyle de l'aldéhyde aliphatique en C11-C18 sont préférés.

Selon un mode de réalisation, l'étape d'acétalisation ou de trans-acétalisation peut être effectuée avec ou sans solvant. Lorsque la réaction est effectuée en présence d'un solvant, le solvant est de préférence un solvant polaire.

Typiquement, le solvant peut être choisi parmi le diméthylformamide (DMF), le diméthylsulfoxyde (DMSO), le diméthylacétamide (DMA), l'acétonitrile (CH₃CN), le tétrahydrofurane (THF), le 2-méthyltétrahydrofuranne (2Me-THF), l'éther méthylique de cyclopentyle (CPME), le methanol (MeOH), l'éthanol (EtOH), le propanol (PrOH), l'isopropanol (iPrOH), le butanol (BuOH), l'éther dibutylique (DBE), le méthyle tert-butyle éther (MTBE) et le triméthoxypropane (TMP).

Des travaux expérimentaux approfondis ont conduit à une sélection de conditions permettant l'observation de taux de conversion et de rendements optimaux au cours des étapes d'acétalisation ou de trans-acétalisation. Les meilleurs résultats ont été obtenus lorsque le rapport molaire [(aldéhyde aliphatique en C11-C18 ou leur acétal) : désoxyhexose ou son dérivé] est compris entre 5:1 et 1:5, de préférence entre 4:1 et 1:4, et de manière avantageuse entre 3:1 et 1:3.

Les inventeurs ont montré plus particulièrement, que lors d'une réaction d'acétalisation, le rapport molaire aldéhyde aliphatique en C11-C18: désoxyhexose ou son dérivé compris entre 1:1 et 1:5 , de préférence entre 1:1 et 1:4, et de manière préférée entre 1:3 et 1:2 améliore les rendements et fournit des taux optimaux de conversion.

Les inventeurs ont montré en outre qu'au cours des réactions de trans-acétalisation, un rapport molaire acétal aliphatique en C11-C18 : (désoxyhexose ou dérivé de désoxyhexose) compris entre 1:1 et 5:1, de préférence entre 5:4 et 4:1, de préférence entre 3:1 et 4:3, de préférence encore entre 3:2 et 2:5 améliore les rendements et fournit des taux optimaux de conversion. Les catalyseurs utilisés sont les mêmes que lors de la réaction d'acétalisation.

Selon un mode de réalisation, le procédé de l'invention comprend en outre au moins une étape de neutralisation et/ou de filtration et/ou de purification après l'une quelconque des étapes de déshydratation le cas échant, d'acétalisation ou de trans-acétalisation.

Lorsqu'une étape de purification est prévue, ladite étape de purification peut être par exemple, une cristallisation, une recristallisation ou une chromatographie. De préférence, la chromatographie est réalisée en utilisant un solvant polaire non-aqueux. En général, quand une étape de filtration et/ou de purification est prévue avant l'étape d'hydrogénolyse, le solvant polaire non-aqueux peut être identique à celui utilisé lors de l'étape d'hydrogénolyse.

Avantageusement, l'étape d'hydrogénolyse est effectuée à une température comprise entre 80°C et 140°C, et/ou à une pression d'hydrogène comprise entre 15 et 50 bar, de préférence entre 20 et 40 bar.

L'étape d'hydrogénolyse est effectuée avantageusement dans un solvant polaire aprotique, de préférence un solvant non-aqueux. En effet, les solvants aprotiques offrent une meilleure conversion. Des exemples de solvants aprotiques sont, entre autres, sans limitation, les alcanes, le 1,2,3-triméthoxypropane (TMP), le tert-butyle éther de méthyle (MTBE), le tétrahydrofurane (THF), le 2-méthyl tétrahydrofuranne (2Me-THF), l'éther de dibutyle (DBE) et le cyclopentylméthyléther (CPME). De préférence, le solvant aprotique est le CPME. Les alcanes sont avantageux car ils permettent une meilleure solubilisation de l'hydrogène dans le milieu. Cependant, la conversion est moins élevée que d'autres solvants aprotiques tels que le CPME. En général, parmi les alcanes, le dodécane et l'heptane sont préférés.

L'étape d'hydrogénolyse est effectuée de préférence dans un solvant aprotique polaire à une température comprise entre 80°C et 140°C et/ou sous une pression d'hydrogène comprise entre 15 et 50 bar, en présence d'un catalyseur adapté aux réactions d'hydrogénolyse.

De préférence, l'étape d'hydrogénolyse est effectuée dans un solvant polaire non-aqueux à une température comprise entre 100°C et 130°C et/ou à une pression comprise entre 25 et 35 bar.

Généralement, l'hydrogénolyse est effectuée en présence d'un catalyseur adapté tel qu'un catalyseur à base de métaux précieux ou de métaux communs. Plus particulièrement, les métaux communs peuvent être des métaux ferreux ou non-ferreux. Typiquement, l'hydrogénolyse est effectuée en présence d'un catalyseur à base de métaux ferreux.

A titre indicatif, un catalyseur de métal appartenant au groupe des métaux ferreux peut être du nickel, du cobalt ou du fer.

De préférence, l'hydrogénolyse est réalisée en utilisant un catalyseur à base de métaux précieux tels que le palladium, le rhodium, le ruthénium, le platine ou l'iridium.

En règle générale, le catalyseur utilisé au cours de l'hydrogénolyse peut être fixé sur un support tel que le carbone, l'alumine, la zircone ou la silice ou un mélange quelconque de ceux-ci. Un tel support est par exemple une bille. Ainsi, un catalyseur de palladium fixé sur des billes de charbon (Pd / C) peut être avantageusement utilisé. Ces catalyseurs peuvent être dopés par l'adjonction de métaux précieux ou métaux communs. On parle d'agent de dopage. Typiquement, l'agent de dopage représente 1 à 10% en poids du catalyseur.

L'invention concerne également une composition bactéricide ou bactériostatique comprenant un mélange d'isomères de position de monoéthers ou de monoacétals d'alkyle de désoxyhexose présentant un radical éther alkyle ou acétal alkyle sur 2 positions distinctes du désoxyhexose ou du dérivé de désoxyhexose ainsi que les sels pharmaceutiquement acceptables de ceux-ci dans lequel le groupe alkyle comprend entre 11 à 18 atomes de carbone, préférentiellement de 11 à 13 atomes de carbone. Ledit radical acétal alkyle ou éther alkyle étant en position 2-O-, 3-O-, et/ou 4-O-.

Le terme "sels pharmaceutiquement acceptables" désigne n'importe quel sel qui, par administration au patient est capable de fournir (directement ou indirectement) un composé comme celui décrit présentement. La préparation de sels peut être effectuée par des procédés connus dans l'état de la technique.

Par « isomères » on entend, des entités moléculaires qui ont la même composition atomique (formule moléculaire), mais des formules linéaires différentes ou des formules stéréochimiques différentes (PAC, 1994, 66, 1077 (Glossary of terms used in physical organic chemistry (IUPAC Recommendations 1994)) page 1129

Les isomères sont des régioisomères et/ou des diastéréoisomères.

Selon la présente invention, le terme « diastéréoisomère » se réfère à des stéréo-isomères (isomères qui possèdent une constitution identique, mais qui diffèrent dans l'arrangement de leurs atomes dans l'espace) qui ne sont pas des énantiomères (des entités moléculaires ayant des formules stéréochimiques formant des images dans le miroir non superposables).

Selon la présent invention, le terme « régioisomère » ou l'expression « isomère de position » se réfère à des isomères dont un groupe fonctionnel est placé sur des carbones différents de la chaîne carbonée. Plus particulièrement, on entend des isomères de monoéthers ou de monoacétals d'alkyle de désoxyhexose ou de dérivé de désoxyhexose dans lesquels le radical monoéther ou monoacétal alkyl est positionné sur différents oxygènes présents sur le squelette du désoxyhexose ou du dérivé de désoxyhexose.

Typiquement, on peut citer par exemple le 2,3-*O*-dodécylidene α-L-rhamnopyranoside de méthyle et/ou le 2-*O*-dodécyl α-L-rhamnopyranoside de méthyle et/ou le 3-*O*-dodécyl α-L-rhamnopyranoside de méthyle

Typiquement, la composition est bactéricide ou bactériostatique vis-à-vis des bactéries à Gram positif. L'invention porte également, sur l'utlisation d'une telle composition comme agent bactéricide ou bactériostatique vis-à-vis des bactéries à Gram positif.

Avantageusement, la composition bactéricide ou bactériostatique ou l'agent bactéricide ou bactériostatique est incorporé(e) dans une composition alimentaire, cosmétique, pharmaceutique, phytosanitaire, vétérinaire ou de traitement de surface. Telle que par exemple, une composition cosmétique et/ou dermatologique de nettoyage et/ou de soin pour la peau, en particulier sous la forme d'une crème, un gel, une poudre, une lotion, un beurre notamment, un gel douche, savon, shampoing, bain de douche, déodorant, anti-transpirant, lingette humide, formulation d'écran solaire ou formulation cosmétique décorative.

L'invention concerne également, une composition caractérisée en ce qu'elle comprend un acétal d'alkyle ou un éther d'alkyle de désoxyhexose ou d'un dérivé de désoxyhexose dans lequel le groupe alkyle comprend entre 11 à 18 atomes de carbone, un sel pharmaceutiquement acceptable, un isomère ou un mélange d'isomères de celui-ci, ledit dérivé de désoxyhexose étant un désoxyhexose glycosylé et/ou hydrogéné et/ou déshydraté, pour son utilisation comme agent bactéricide ou bactériostatique, ledit radical acétal alkyle ou éther alkyle est en position 2-*O*, 3-*O*-, 4-*O*-, les isomères étant préférentiellement choisis parmi les régioisomères et/ou les diastéréoisomères.

L'invention concerne en outre, une composition caractérisée en ce qu'elle comprend un acétal d'alkyle ou un éther d'alkyle de désoxyhexose ou d'un dérivé de désoxyhexose dans lequel le groupe alkyle comprend entre 11 à 18 atomes de carbone, un sel pharmaceutiquement acceptable, un isomère ou un mélange d'isomères de celui-ci, ledit dérivé de désoxyhexose étant un désoxyhexose glycosylé et/ou hydrogéné et/ou déshydraté, pour son une utilisation comme produit d'hygiène ou dermatologique à usage externe.

Typiquement un « produit d'hygiène » se réfère à tout produit utilisé pour le nettoyage, la désinfection ou l'hygiène, y compris par exemple une lotion, mousse, spray et liquide mais également des lingettes ou tout support susceptible d'être imprégné de la composition selon l'invention. L'expression « produit dermatologique » se réfère à tout produit destiné à une application sur la peau ou les muqueuses.

### Utilisation dans le traitement ou la prévention d'une infection à bactéries Gram positif.

L'invention concerne de plus, une composition selon l'invention pour une utilisation dans le traitement ou à la prévention des infections bactériennes par des bactéries à Gram positif.

Par « traitement », on entend traitement à titre curatif (visant à au moins réduire, éradiquer ou stopper le développement de l'infection) chez un patient. Par « prévention », on entend traitement à titre prophylactique (visant à réduire le risque d'apparition de l'infection) chez un patient.

Le «patient» peut-être, par exemple un être humain ou un mammifère non-humain (par exemple un rongeur (souris, rat), un félin, un chien ou un primate) affecté par ou susceptible d'être affecté par des infections bactériennes et notamment à Gram positif. De préférence, le sujet est un humain.

L'expression « Gram-positif » se réfère à des bactéries qui sont colorées en bleu foncé ou le violet par la coloration de Gram, par opposition aux bactéries gram-négatives qui ne peuvent pas retenir le colorant violet. La technique de coloration est bien connue de l'homme du métier et repose sur les caractéristiques membranaires et de paroi de la bactérie.

Typiquement, les bactéries à Gram positif sont des bactéries de l'embranchement des *Firmicutes*, typiquement de la classe des *Bacilli* notamment choisies parmi les bactéries de l'ordre des *Lactobacillales* ou des *Bacillales.*

Selon une forme de réalisation de l'invention, les bactéries de l'ordre des *Bacillales* sont choisies parmi la famille des *Alicyclobacillaceae*, *Bacillaceae*, *Caryophanaceae*, *Listeriaceae*, *Paenibacillaceae*, *Pasteuriaceae*, *Planococcaceae*, *Sporolactobacillaceae*, *Staphylococcaceae*, *Thermoactinomycetacea* et *Turicibacteraceae*
Typiquement, les bactéries de la famille des *Listeriaceae* sont par exemple du genre des *Brochothrix* ou des *Listeria* et peuvent être typiquement, choisies parmi *L*. *fleischmannii, L. grayi, L. innocua, L. ivanovii, L. marthii, L. monocytogenes, L. rocourtiae, L. seeligeri, L. weihenstephanensis et L. welshimeri.*

Lorsque les bactéries à Gram positif sont des bactéries de la famille des *Staphylococcaceae*, elles sont notamment choisies parmi les bactéries du genre des *Staphylococcus, Gemella, Jeotgalicoccus, Macrococcus, Salinicoccus et Nosocomiicoccus.*

Les bactéries du genre des *Staphylococcus* par exemple choisies parmi *S. arlettae, S. agnetis, S. aureus, S. auricularis, S. capitis, S. caprae, S. carnosus, S. caseolyticus, S. chromogenes, S. cohnii, S. condimenti, S. delphini, S. devriesei, S. epidermidis, S. equorum, S. felis, S. fleurettii, S. gallinarum, S. haemolyticus, S. hominis, S. hyicus, S. intermedius, S. kloosii, S. leei, S. lentus, S. lugdunensis, S. lutrae, S. massiliensis, S. microti, S. muscae, S. nepalensis, S. pasteuri, S. pettenkoferi, S. piscifermentans, S. pseudintermedius, S. pseudolugdunensis, S. pulvereri, S. rostri, S. saccharolyticus, S. saprophyticus, S. schleiferi, S. sciuri, S. simiae, S. simulans, S. stepanovicii, S. succinus, S. vitulinus, S. warneri et S. xylosus.*

Selon une autre forme de réalisation de l'invention, les bactéries de l'ordre des *Lactobacillales* sont choisies parmi une famille des *Aerococcaceae, Carnobacteriaceae, Enterococcaceae, Lactobacillaceae, Leuconostocaceae et Streptococcaceae.*

Typiquement, les bactéries de la famille des *Enterococcaceae* sont choisies parmi les bactéries du genre des *Bavariicoccus, Catellicoccus, Enterococcus, Melissococcus, Pilibacter, Tetragenococcus, Vagococcus.*

Les bactéries du genre des *Enterococcus* sont choisies par exemple, parmi *E. malodoratus, E. avium, E. durans, E. faecalis, E. faecium, E. gallinarum, E. hirae, E. solitarius, préférentiellement, E. avium, E. durans, E. faecalis et E. faecium.*

Les bactéries du genre des *Staphylococcus* et plus particulièrement *S. aureus* sont responsables de nombreuses infections de la peau ou des muqueuses telles que la muqueuse vaginale ou nasale. Par exemple, des infections telles que la folliculite, les abcès, les panaris, les furoncles, l'impetigo, les infections interdigitales, l'anthrax (anthrax staphylococcique), les cellulites, les surinfections de plaies, les otitis sinusitis, l'hydradénite, les mastites infectieuses, les infections post-traumatiques de la peau ou les infections de la peau brûlée.

Les bactéries du genre des *Enterococcus* et notamment *E. faecalis* sont responsables notamment d'endocardites, d'infections de la vessie, de la prostate ou de l'épididyme

### Méthode non-thérapeutique de désinfection ou de prévention de la colonisation bactérienne d'un substrat

L'invention porte en outre, sur une méthode non-thérapeutique de désinfection ou de prévention de la colonisation bactérienne par des bactéries à Gram positif d'un substrat comprenant la mise en contact du substrat avec une composition selon l'invention.

Typiquement, le substrat est tout support susceptible d'être colonisé par des bactéries à Gram positif et susceptible de transmettre l'infection à un animal par contact ou par ingestion.

Par exemple, le substrat peut être un aliment d'origine végétale ou animale ou une composition alimentaire comprenant de tels aliments ou un extrait de ces aliments et notamment des céréales, des fruits, des légumes, de la viande, du poisson, des abats.

Le substrat peut être également un ou plusieurs éléments sélectionnés parmi des métaux, des plastiques, du verre, du béton, de la pierre.

Préférentiellement le substrat est un ustensile, un outil ou un appareil utilisé dans l'industrie alimentaire, (ustensiles de cuisine, contenant, système de conservation à froid, réfrigérateur, chambres froides..) en milieu hospitalier, tels que par exemple des outils de chirurgie ou des prothèses ou dans les transports en commun (barre de maintien transport en commun, sièges...).

Autrement dit, l'invention concerne une composition pour la désinfection, l'épuration, la stérilisation ou la purification des surfaces, telles que définies ci-dessous.

Bien qu'ayant des significations distinctes, les termes « comprenant », « contenant », « comportant » et « consistant en » ont été utilisés de manière interchangeable dans la description de l'invention, et peuvent être remplacés l'un par l'autre.

L'invention sera mieux comprise à la lecture des figures et exemples suivants donnés uniquement à titre d'exemple.

### Exemples

Les acétals de glycopyranoside de méthyle ont été préparés par acétalisation ou transacétalisation des sucres suivant la procédure préalablement décrite dans le brevet N°13/01375 « Procédé pour la préparation d'acétals cycliques alkyl à longues chaînes, à base de sucres ». Les acétals alkyle de glycopyranoside de méthyle sont ensuite réduits utilisant des conditions de réduction sans catalyseur acide préalablement décrite dans le brevet N°14/01346. A titre indicatif, la synthèse d'acétals et d'éthers de glycopyranoside de méthyle est détaillée ci-dessous.

### EXEMPLE 1 : Procédure générale pour la préparation d'acétals alkyle de glycopyranoside de méthyle (A)

Dans un ballon de 100 mL, sous atmosphère d'argon, le glycopyranoside de méthyle (2 équivalent) est dissout dans le THF sec (10 mL) en présence de sulfate de sodium (1,5 équivalent). L'aldéhyde (1 équivalent) est additionné goutte-à-goutte sur une durée d'une minute, suivi de l'Amberlyst 15 (20% en masse par rapport à l'aldéhyde). Le mélange réactionnel est agité magnétiquement au reflux (65°C) pendant 3 heures. Après retour à température ambiante, le milieu réactionnel est filtré, lavé avec l'acétate d'éthyle (2x25 mL) et le filtrat est concentré sous pression réduite. Le résidu est purifié par chromatographie sur colonne de gel de silice (AcOEt/cyclohexane) pour donner les acétals alkyle de glycopyranoside de méthyle.

### Exemple comparatif 1a :

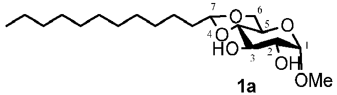

**4,6-*O*-Dodécylidene α-D-glucopyranoside de méthyle (1a):** Le composé **1a** a été préparé à partir de l'a-D-glucopyranoside de méthyle (3.22 g, 16.6 mmol) et du dodécanal (1.52 g, 8.3 mmol) suivant la procédure **(A).** Après réaction, le résidu a été purifié par chromatographie sur colonne de gel de silice (EtOAc/cyclohexane 60 : 40) pour donner **1a** (0.77 g, 26%) sous la forme d'un solide blanc. Point de fusion = 69°C; RMN ¹H (300 MHz, CDCl₃) δ_{H}: 0.86 (3H, t, *J* = 7, CH₃), 1.17-1.32 (16H, m, 8CH₂), 1.33-1.47 (2H, m, CH₂), 1.53-1.74 (2H, m, CH₂), 2.64 (2H, br s, OH³+OH²), 3.24 (1H, t, *J* = 9.0, CH³), 3.41 (3H, s, OCH₃), 3.49-3.68 (3H, m, CH⁵+CH⁶+CH²), 3.84 (1H, t, *J* = 9.0, CH⁴), 4.10 (1H, dd, *J* = 10.0 et 5.0, CH⁶), 4.52 (1H, t, *J* = 5.0, CH⁷), 4.74 (1H, d, *J* = 4.0, CH¹); RMN ¹³C (75 MHz, CDCl₃) δ_{C}: 14.24 (CH₃), 22.80 (CH₂), 24.20 (CH₂), 29.46 (CH₂), 29.58 (CH₂), 29.62 (CH₂), 29.67 (CH₂), 29.74 (CH₂), 29.76 (CH₂), 32.03 (CH₂), 34.36 (CH₂), 55.57 (OCH₃), 62.63 (CH⁵), 68.57 (CH₂⁶), 71.81 (CH⁴), 73.02 (CH²), 80.46 (CH³), 99.85 (CH¹), 102.84 (CH⁷); IR vₘₐₓ: 3388 (OH), 2921, 2852, 1466, 1378, 1089, 1063, 1037, 991; HRMS (ESI⁺) calculé pour C₁₉H₃₆NaO₆: 383.2404 [M+Na]⁺; mesuré : 383.2398 (+1.6 ppm); Rf = 0,30 (EtOAc/cyclohexane 60:40).

### Exemple comparatif 1b :

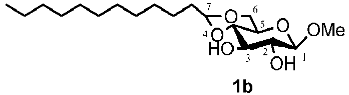

**4,6-*O*-Dodécylidene β-D-glucopyranoside de méthyle (1b):** Le composé **1b** a été préparé à partir du β-D-glucopyranoside de méthyle (5.00 g, 25.7 mmol) et du dodécanal (2.37 g, 12.8 mmol) suivant la procédure **(A).** Après réaction, le résidu a été purifié par chromatographie sur colonne de gel de silice (EtOAc/cyclohexane, de 30:70 à 50:50) pour donner **1b** (1.30 g, 28%) sous la forme d'un solide blanc. Point de fusion = 84°C; RMN ¹H (300 MHz, CDCl₃) δ_{H}: 0.87 (3H, t, *J* = 6.7, CH₃), 1.25 (16H, app br s, 8 CH₂), 1.34-1.45 (2H, m, CH₂), 1.53-1.73 (2H, m, CH₂), 3.25-3.34 (2H, m, CH²+CH⁵), 3.44 (1H, dd, *J* = 9.0, 7.0, CH³), 3.56 (4H, s, CH₂⁶+ OCH₃), 3.73 (1H, m, CH⁴), 4.18 (1H, dd, *J* = 10.4, 4.4, CH₂⁶), 4.28 (1H, d, *J* = 7.7, CH¹), 4.54 (1H, t, *J* = 5.1, CH⁷); RMN ¹³C (75 MHz, CDCl₃) δ_{C}: 14.13 (CH₃), 22.70 (CH₂), 24.14 (CH₂), 29.35 (CH₂), 29.45 (CH₂), 29.50 (CH₂), 29.56 (CH₂), 29.63 (CH₂), 29.65 (CH₂), 31.92 (CH₂), 34.23 (CH₂), 55.51 (OCH₃), 66.21 (CH⁵), 68.21 (CH₂⁶), 73.19 (CH⁴), 74.61 (CH²), 80.00 (CH³), 102.83 (CH⁷), 104.07 (CH¹); IR vₘₐₓ: 3650 (OH), 2950, 2824, 2867, 2159, 2028, 1112; HRMS (ESI⁺) calculé pour C₁₉H₃₆NaO₆ : 383.2404 [M+Na]⁺; mesuré : 383.2395 (+2.3 ppm). Rf = 0,30 (EtOAc/cyclohexane 40:60)

### Exemple comparatif 1c :

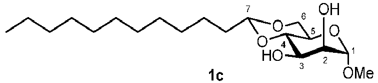

**4,6-*O*-Dodécylidene α-D-mannopyranoside de méthyle (1c):** Le composé **1c** a été préparé à partir de l'a-D-mannopyranoside de méthyle (4.00 g, 20.5 mmol) et du dodécanal (3.45 g, 18.7 mmol) suivant la procédure **(A).** Après réaction, le milieu réactionnel est concentré sous pression réduite et dissout dans le CH₂Cl₂. La phase organique est lavée à l'eau (3 x 100 mL), avec une solution de NaCl saturée (2 x 100 mL), séchée (Na₂SO₄) et concentrée sous pression réduite. Le résidu a été purifié par chromatographie sur colonne de gel de silice (EtOAc/cyclohexane, de 20:80 à 50:50) pour donner 1c (0.73 g, 11%) sous la forme d'un solide blanc. Point de fusion = 104°C; RMN ¹H (300 MHz, CDCl₃) δ_{H}: 0.88 (3H, t, *J* = 6.9, CH₃), 1.17-1.32 (16H, m, 8 CH₂), 1.37-1.42 (2H, m, CH₂), 1.58-1.68 (2H, m, CH₂), 3.37 (3H, s, OCH₃), 3.53-3.72 (3H, m, CH³+CH⁵+CH⁶), 3.98 (1H, dd, *J* = 9.0, 3.7, CH₂), 4.13 (1H, dd, *J* = 3.6, 1.4, CH⁴), 4.58 (1H, dd, *J* = 8.8, 2.9, CH⁶), 4.10 (1H, t, *J* = 5.1, CH⁷), 4.73 (1H, d, *J* = 1.3, CH¹); RMN ¹³C (75 MHz, CDCl₃) δ_{C}: 14.13 (CH₃), 22.69 (CH₂), 24.10 (CH₂), 29.35 (CH₂), 29.46 (CH₂), 29.51 (CH₂), 29.56 (CH₂), 29.63 (CH₂), 29.65 (CH₂), 31.92 (CH₂), 34.40 (CH₂), 55.05 (OCH₃), 63.00 (CH⁵), 68.38 (CH₂⁶), 68.81 (CH²), 70.82 (CH⁴), 78.23 (CH³), 101.15 (CH¹), 103.06 (CH⁷); IR vₘₐₓ: 3380 (OH), 2924, 2852, 1466, 1156, 1029, 682; HRMS (ESI⁺) calculé pour C₁₉H₃₆NaO₆: 383.2404 [M+Na]⁺; mesuré : 383.2396 (+2.2 ppm). Rf = 0,2 (cyclohexane/EtOAc, 70:30).

### Exemple comparatif 1d :

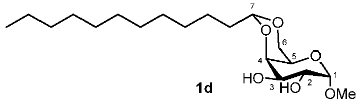

**4,6-*O*-Dodécylidene α-D-galactopyranoside** de **méthyle (1d):** Le composé **1d** a été préparé à partir de l'a-D-galactopyranoside de méthyle (5.00 g, 25.7 mmol) et du dodécanal (2.37 g, 12.9 mmol) suivant la procédure **(A).** Après réaction, le milieu réactionnel est concentré sous pression réduite pour donner **1d** (2.30 g, 45%) sous la forme d'un solide blanc sans purification par chromatographie. Point de fusion = 115°C; RMN ¹H (300 MHz, CDCl₃) δ_{H}: 0.89 (3H, t, *J* = 6.7, CH₃), 1.15-1.50 (18H, m, 9 CH₂), 1.61-1.71 (2H, m, CH₂), 3.45 (3H, s, OCH₃), 3.61 (1H, app. s, CH⁵), 3.77-3.94 (3H, m, CH⁴+CH²CH⁶), 4.04 (1H, d, *J* = 2.5, H³), 4.14 (1H, dd, *J* = 12.5, 1.4, CH⁶), 4.59 (1H, t, *J* = 5.2, CH⁷), 4.91 (1H, d, *J* = 3.2, CH¹); RMN ¹³C (75 MHz, CDCl₃) δ_{C}: 14.06 (CH₃), 22.50 (CH₂), 23.49 (CH₂), 29.27 (CH₂), 29.34 (CH₂), 29.41 (CH₂), 29.48 (CH₂), 29.55 (CH₂), 29.61 (CH₂), 31.97 (CH₂), 34.47 (CH₂), 55.66 (OCH₃), 62.45 (CH⁵), 68.92 (CH₂⁶), 69.82 (CH₂), 69.92 (CH⁴), 75.42 (CH³), 100.1 (CH⁷), 102.1 (CH¹); IR vₘₐₓ: 3414, 3328 (OH), 2916, 2850, 2160, 1121, 1032; HRMS (ESI⁺) calculé pour C₁₉H₃₆NaO₆ 383.2404 [M+Na]⁺; mesuré : 383.2389 (+4.0 ppm). Rf = 0,6 (EtOAc/cyclohexane, 60:40).

### Exemple 1e :

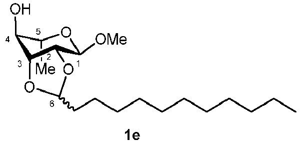

**2,3-*O*-dodécylidene α-L-rhamnopyranoside de méthyle (1e):** Le composé **1e** a été préparé à partir de l'a-L-rhamnopyranoside de méthyle (1.00 g, 5.60 mmol) et du dodécanal (0.94 g, 5.10 mmol) suivant la procédure **(A).** Après réaction, le milieu réactionnel est concentré sous pression réduite et dissout dans le CH₂Cl₂. La phase organique est lavée à l'eau (3 x 100 mL), avec une solution de NaCl saturée (2 x 100 mL), séchée (Na₂SO₄) et concentrée sous pression réduite. Le résidu a été purifié par chromatographie sur colonne de gel de silice (EtOAc/cyclohexane, de 0:100 puis de 10:90 à 100:0). Un mélange inséparable 53:47 de deux diastéréoisomères de **1e** (0.85 g, 49%) a été obtenu sous la forme d'un solide beige. Point de fusion = 46°C; RMN ¹H (300 MHz, CDCl₃) δ_{H} pour le mélange de diastéréoisomères : 0.88 (6H, t, *J* = 6.7, 2xCH₃), 1.25-1.32 (42H, m, 18(CH₂) + 2xCH₃), 1.57-1.63 (2H, m, CH₂), 1.67-1.74 (2H, m, CH₂), 3.34-3.42 (2H, m, 2 x CH⁴), 3.38 (3H, s, OCH₃), 3.39 (3H, s, OCH₃), 3.65-3.67 (2H, m, 2 x CH⁵), 3.93-4.00 (2H, m, CH² + CH³), 4.00-4.08 (1H, m, CH²), 4.18 (1H, dd, *J* = 7.4, 5.4, CH³), 4.85 (1H, s, CH¹), 4.88 (1H, s, CH¹), 4.98 (1H, t, *J* = 5.0, CH⁶), 5.24 (1H, t, *J* = 4.8, CH⁶); RMN ¹³C (75 MHz, CDCl₃) δ_{C} pour le mélange de diastéréoisomères : 14.13 (2 x CH₃), 17.41 (CH₃), 17.56 (CH₃), 22.70 (2 CH₂), 23.77 (CH₂), 24.23 (CH₂), 29.35 (2 CH₂), 29.49 (CH₂), 29.50 (CH₂), 29.53 (2 CH₂), 29.56 (2 CH₂), 29.62 (2 CH₂), 29.65 (2 CH₂), 31.92 (2 CH₂), 34.90 (CH₂), 35,41 (CH₂), 54.96 (2 OCH₃), 65.24 (CH⁵), 65.87 (CH⁵), 71.57 (CH⁴), 74.94 (CH⁴), 75.17 (CH³), 77.37 (CH²), 77.40 (CH²), 78.99 (CH³), 97.96 (CH¹), 98.28 (CH¹), 104.2 (CH⁶), 104.3 (CH⁶); IR vₘₐₓ: 3650, 3238 (OH), 2921, 2852, 2159, 2029, 1136, 1029; HRMS (ESI⁺) calculé pour C₁₉H₃₆NaO₅ 367.2455 [M+Na]⁺; mesuré : 367.2452 (+0.9 ppm). Rf = 0,5 (EtOAc/cyclohexane, 50:50).

### EXEMPLE 2 : Procédure générale pour la préparation de mélange de régioisomères d'éthers alkyle de glycopyranoside de méthyle (B)

Dans un autoclave de 100 mL en acier inoxydable, l'acétal alkyle de glycopyranoside de méthyle (3 mmol) est dissout dans le cyclopentylméthyléther (CPME, 30 mL) et du 5%-Pd/C (0,45 g, 5% molaire en palladium) est ensuite ajouté. Le réacteur est fermé hermétiquement, purgé trois fois avec de l'hydrogène et l'hydrogène est introduit à une pression de 30 bars. Le mélange réactionnel est agité mécaniquement et est chauffé à 120°C pendant 15 heures. Après refroidissement à température ambiante, la pression d'hydrogène est libérée et le mélange réactionnel est dilué dans l'éthanol absolu (100 mL) et filtré (filtre Millipore Durapore 0,01 um). Le filtrat est concentré sous pression réduite pour donner le mélange de régioisomères d'éthers alkyle de glycopyranoside de méthyle.

### Exemple comparatif 2a :

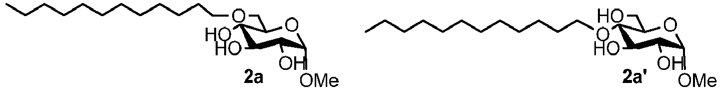

**6-*O*-Dodécyl α-D-glucopyranoside de méthyle (2a) et 4-*O*-dodécyl α-D-glucopyranoside de méthyle (2a'):** Les composés **2a** et **2a'** ont été préparés à partir du 4,6-*O*-dodécylidene α-D-glucopyranoside de méthyle **1a** (5.00 g, 14 mmol) suivant la procédure générale **(B).** Un mélange 73:27 de **2a** et **2a'** (2.52 g, 51%) a été obtenu sous forme d'un solide blanc. Pour faciliter la caractérisation des composés, les régioisomères du mélange peuvent être séparés par chromatographie sur colonne de gel de silice (EtOAc/cyclohexane, de 50:50 à 100:0 puis EtOH/EtOAc 10:90). **2a:** Solide blanc. RMN ¹H (300 MHz, CDCl₃) δ_{H}: 0.87 (3H, t, *J* = 7, CH₃ alkyl), 1.09-1.44 (18H, m, 9(CH₂) alkyl), 1.47-1.70 (2H, m, CH₂ alkyl), 3.41 (3H, s, OCH₃), 3.43-3.84 (7H, m), 4.21 (3H, br s, OH), 4.74 (1H, d, *J* = 4, CH-anomérique); RMN ¹³C (75 MHz, CDCl₃) δ_{C}: 14.25 (CH₃), 22.82 (CH₂), 26.17 (CH₂), 29.50 (CH₂), 29.67 (CH₂), 29.73 (CH₂), 29.77 (CH₂), 29.80 (2CH₂), 29.83 (CH₂), 32.06 (CH₂), 55.35 (OCH₃), 70.33 (CH), 70.51 (CH₂), 71.23 (CH), 72.10 (CH), 72.30 (CH₂), 74.49 (CH), 99.57 (CH); IR vₘₐₓ: 3402 (OH), 2918, 2851, 1467, 1370, 1057, 1015, 902; HRMS (ESI⁺) calculé pour C₁₉H₃₈NaO₆: 385.2561 [M+Na]⁺; mesuré : 385.2558 (+0.6 ppm); Rf = 0,16 (EtOAc/EtOH 10:1). **2a':** solide blanc. RMN ¹H (300 MHz, CDCl₃) δ_{H}: 0.87 (3H, t, *J* = 7, CH₃ alkyl), 1.14-1.42 (18H, m, 9(CH₂) alkyl), 1.47-1.71 (2H, m, CH₂ alkyl), 2.16 (3H, br s, OH), 3.24 (1H, t, *J* = 10); 3.41 (3H, s, OCH₃), 3.49 (1H, dd, *J* = =10 et 4), 3.54-3.66 (2H, m), 3.69-3.91 (4H, m), 4.74 (1H, d, *J* = 4, CH-anomérique); RMN ¹³C (75 MHz, CDCl₃) δ_{C}: 14.26 (CH₃), 22.83 (CH₂), 26.20 (CH₂), 29.49 (CH₂), 29.64 (CH₂), 29.74 (2CH₂), 29.77 (CH₂), 29.80 (CH₂), 30.47 (CH₂), 32.06 (CH₂), 55.46 (OCH₃), 62.15 (CH₂), 70.99 (CH), 72.81 (CH), 73.28 (CH₂), 75.05 (CH), 77.94 (CH), 99.20 (CH); IR vₘₐₓ: 3295 (OH), 2913, 2848, 1739, 1469, 1370, 1114, 1067, 1042, 993; HRMS (ESI⁺) calculé pour C₁₉H₃₈NaO₆: 385.2561 [M+Na]⁺; mesuré : 385.2574 (-3.5 ppm); Rf = 0,24 (EtOAc/EtOH 10:1).

### Exemple comparatif 2b :

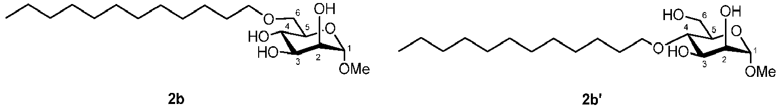

**6-*O*-Dodécyl α-D-mannopyranoside de méthyle (2b) et 4-*O*-dodécyl α-D-mannopyranoside de méthyle (2b'):** Les composés **2b** et **2b'** ont été préparés à partir du 4,6-*O*-dodécylidene α-D-mannopyranoside de méthyle 1c (0.70 g, 1.94 mmol) suivant la procédure générale **(B).** Après réaction, le résidu a été purifié par chromatographie sur colonne de gel de silice (EtOAc/cyclohexane, 40:60). Un mélange inséparable 75:25 de **2b** et **2b'** (0.24 g, 34%) a été obtenu sous forme d'une huile incolore. RMN ¹H (300 MHz, CDCl₃) δ_{H} pour le régioisomère majoritaire **2b** : 0.88 (3H, t, *J* = 6.7, CH₃), 1.20-1.35 (18H, m, 9 CH₂), 1.55-1.61 (2H, m, CH₂), 3.35 (3H, s, OCH₃), 3.44-3.57 (2H, m, OCH₂), 3.60-3.98 (6H, m, CH²+CH³+CH⁴+CH⁵+CH₂⁶), 4.73 (1H, d, *J* = 1.5, CH¹); RMN ¹³C (75 MHz, CDCl₃) δ_{C} pour le régioisomère majoritaire **2b** : 14.06 (CH₃), 22.63 (CH₂), 25.95 (CH₂), 29.30 (CH₂), 29.42 (CH₂), 29.44 (CH₂), 29.54 (CH₂), 29.57 (CH₂), 29.58 (CH₂), 29.61 (CH₂), 31.86 (CH₂), 54.96 (OCH₃), 69.50 (CH⁵), 69.65 (CH⁴), 70.37 (CH²), 71.12 (CH₂⁶), 71.67 (CH₃), 72.14 (OCH₂), 100.7 (CH¹); IR vₘₐₓ: 3650, 3238 (OH), 2921, 2852, 2159, 2029, 1976, 1156; HRMS (ESI⁺) calculé pour C₁₉H₃₈NaO₆: 385.2561 [M+Na]⁺; mesuré : 385.2555 (+1.5 ppm); Rf = 0,22 (cyclohexane/EtOAc, 60:40).

### Exemple comparatif 2c :

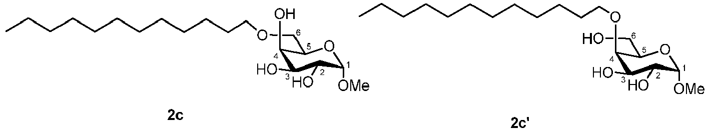

**6-*O*-Dodécyl α-D-galactopyranoside de méthyle (2c) et 4-*O*-dodécyl α-D-galactopyranoside de méthyle (2c'):** Les composés **2c** et **2c'** ont été préparés à partir du 4,6-*O*-dodécylidene α-D-galactopyranoside de méthyle **1d** (0.69 g, 1.90 mmol) suivant la procédure générale **(B).** Après réaction, le résidu a été purifié par chromatographie sur colonne de gel de silice (EtOAc/cyclohexane, 50:50). Un mélange inséparable 90:10 de **2c** et **2c'** (0.19 g, 27%) a été obtenu sous forme d'un solide blanc. Point de fusion = 110°C; RMN ¹H (300 MHz, CDCl₃) δ_{H} pour le régioisomère majoritaire **2c** : 0.87 (3H, t, *J* = 6.6, CH₃), 1.24 (18H, br s, 9 CH₂), 1.55-1.60 (2H, m, CH₂), 3.41 (3H, s, OCH₃), 3.48 (2H, t, *J* = 6.7, OCH₂), 3.67-3.90 (5H, m, 3 CH + CH₂), 4.04-4.05 (1H, m, CH), 4.83 (1H, d, *J* = 3.5, CH¹); RMN ¹³C (75 MHz, CDCl₃) δ_{C} pour le régioisomère majoritaire **2c'** : 14.24 (CH₃), 22.81 (CH₂), 26.17 (CH₂), 29.47 (CH₂), 29.59 (CH₂), 29.61 (CH₂), 29.70 (CH₂), 29.74 (CH₂), 29.76 (2 CH₂), 29.78 (CH₂), 32.44 (CH₂), 55.59 (OCH₃), 69.68 (CH), 70.47 (CH), 71.11 (CH), 71.34 (CH), 72.30 (CH₂), 99.84 (CH¹); IR vₘₐₓ: 3651, 3250 (OH), 2917, 2849, 2493, 2430, 2159, 2029, 1976, 1042; HRMS (ESI⁺) calculé pour C₁₉H₃₈NaO₆: 385.2561 [M+Na]⁺; mesuré : 385.2548 (+3.2 ppm); Rf = 0,30 (cyclohexane/EtOAc, 40:60).

### Exemple 2d :

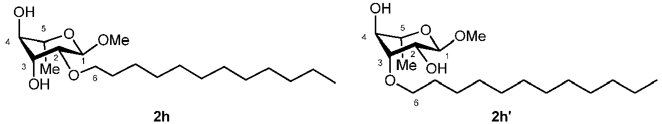

**2-*O*-Dodécyl α-L-rhamnopyranoside de méthyle (2d) et 3-*O*-dodécyl α-L-rhamnopyranoside de méthyle (2d'):** Les composés **2d** et **2d'** ont été préparés à partir du 2,3-*O*-dodécylidene α-L-rhamnopyranoside de méthyle **1e** (0.70 g, 2.03 mmol) suivant la procédure générale **(B).** Après réaction, le résidu a été purifié par chromatographie sur colonne de gel de silice (EtOAc/cyclohexane, 40:60). Un mélange inséparable 93:7 de **2d** et **2d'** (0.19 g, 27%) a été obtenu sous forme d'une huile incolore. RMN ¹H (300 MHz, CDCl₃) δ_{H} pour le régioisomère majoritaire **2d** :0.87 (3H, t, *J* = 6.7, CH₃), 1.18-1.35 (21H, m, 9 (CH₂) + CH₃), 1.53-1.59 (2H, m, CH₂), 2.35 (2H, br s, OH), 3.31-3.47 (5H, m, CH³+CH⁶+OCH₃), 3.52 (1H, dd, *J* = 3.9, 1.3, CH²), 3.54-3.62 (1H, m, CH⁵), 3.62-3.71 (2H, m, CH⁶+CH⁴), 4.71 (1H, app. s, CH¹); RMN ¹³C (75 MHz, CDCl₃) δ_{C} pour le régioisomère majoritaire **2d'** : 14.11 (CH₃), 17.54 (CH₃), 22.68 (CH₂), 25.99 (CH₂), 29.34 (CH₂), 29.41 (CH₂), 29.56 (CH₂), 29.59 (CH₂), 29.62 (CH₂), 29.65 (CH₂), 29.80 (CH₂), 31.91 (CH₂), 54.75 (OCH₃), 67.38 (CH⁵), 71.24 (CH₂), 71.51 (CH⁴), 74.07 (CH³), 78.53 (CH²), 97.83 (CH¹); IR vₘₐₓ: 3650, 3238 (OH), 2921, 2852, 2519, 2029, 2029, 1976, 1070; HRMS (ESI⁺) calculé pour C₁₉H₃₈NaO₅ : 369.2611 [M+Na]⁺; mesuré : 369.2605 (+1.8 ppm); Rf = 0,51 (cyclohexane/EtOAc, 60:40).

### EXEMPLE 3 : Mesure des propriétés bactériostatiques de dérivés acétals et éthers de monosaccharides en C12 sur des bactéries à Gram positifs

Les meilleurs résultats ayant été observés avec des composés ayant un groupement alkyl en C12, des essais ont été effectués sur un plus large panel de souches à gram positif avec des composés obtenus selon les exemples 1 et 2.

### 3.1 Matériels et méthodes

### 3.1.1 Les composés d'intérêt testés :

### Acétals de glucopyranoside de méthyle

- 4,6-*O*-Dodécylidene α-D-glucopyranoside de méthyle (1a)
- 4,6-*O*-Dodécylidene β-D-glucopyranoside de méthyle (1b)

### Mélange d'éthers de glycopyranoside de méthyle

- 6-*O*-Dodécyl α-D-glucopyranoside de méthyle (2a) et 4-*O*-dodécyl α-D-glucopyranoside de méthyle (2a')
- 6-*O*-Dodécyl α-D-mannopyranoside de méthyle (2b) et 4-*O*-dodécyl α-D-mannopyranoside de méthyle (2b')
- 6-*O*-Dodécyl α-D-galactopyranoside de méthyle (2c) et 4-*O*-dodécyl α-D-galactopyranoside de méthyle (2c')
- 2-*O*-Dodécyl α-L-rhamnopyranoside de méthyle (2d) et 3-*O*-dodécyl α-L-rhamnopyranoside de méthyle (2d')

### Mélange d'éthers de sorbitane

- 3-*O*-Dodécyl-1,4-D-sorbitane, 5-*O*-dodécyl-1,4-D-sorbitane et 6-*O*-dodécyl-1,4-D-sorbitane

### 3. 1.2 Les bactéries Gram positif étudiées

Les souches testées sont des souches de référence et des cultures multi résistantes aux antibiotiques, il s'agit de souches cliniques ont été isolées à l'hospice de Lyon et sont les suivantes :
- **Staphylocoques *S*. aureus;** ATCC® 29213™, ATCC 25923,
- Souches de Staphylocoques *S.* aureus résistantes à la méthicilline (Lac-Deleo USA 300), (MU 3), (HT 2004-0012), LY 199-0053, (HT 2002-0417), (HT 2006-1004),
- Souches de Staphylocoques *S.* aureus résistantes à la daptomycine (ST 2015-0188) (ST 2014 1288), (ST 2015- 0989).
- **Enterocoques:** *E. faecalis* (ATCC® 29212™), souches cliniques d'enterocoques *E. faecalis* isolées d'urines : la souche 015206179901 (ci-après 9901), la souche 015205261801 (ci-après 1801)
- **Enterocoques:** *E. faecium* (CIP 103510), souches cliniques *d'*Enterocoques *E. faecium :* Van A 0151850763 (ci-après Van A); la souche 015 205731401 (ci-après 1401),
- **Listeria:** *L. monocytogenes* (CIP 103575), souche cliniques isolée d'hémoculture (015189074801, LM1), souche isolée du liquide céphalo-rachidien (015170199001, LM2), souche clinique isolée d'hémoculture (015181840701, LM3).

### 3.1.3 Préparation de l'inoculum :

Les cultures étudiées, fraîchement isolées (après incubation sur une gélose de sang à 37°C pendant 18h), sont reprises dans de l'eau stérile (10 mL) jusqu'à obtenir une suspension à 0,5 Mac Farland (Mc) soit à 1 à 2 × 10⁸ UFC (bactérie)/cm³. La suspension bactérienne a ensuite été diluée afin d'obtenir une concentration finale de 1 × 10⁶ UFC/cm³.

### 3.1.4 Préparation des plaques multipuits pour la lecture de la CMI :

Chaque puits contient une quantité identique de milieu Mueller-Hinton (milieu riche permettant la culture des bactéries) et de bactérie de 0.5 × 10⁶ UFC/cm³ finale.

Les composés d'intérêt à tester sont solubilisés dans l'éthanol ou le DMSO à 25 mg/mL avant d'être dilués à différentes concentrations de deux en deux. Sur la plaque multi-puits, une première série a été prévue comprenant le milieu de culture sans le composé d'intérêt à tester. Il correspond au témoin de croissance (puits témoins). Ces témoins servent de référence pour comparer la croissance bactérienne avec celles des puits suivants comprenant différentes concentrations du composé d'intérêt à tester. La seconde série de puits comprend la solution mère du composé d'intérêt à tester pour une concentration dans le puit de 256 mg/L (7 mM). Chaque série de puits a été diluée de deux en deux jusqu'à la dernière série pour une concentration finale de 0.25 mg/L (0,0007 mM). Chaque concentration est dupliquée au sein de la même plaque. La plaque est incubée 18h à 37°C. La lecture après incubation montre un trouble dans les puits témoins (révélateur d'une croissance bactérienne). En cas d'activité antibactérienne, la croissance bactérienne est inhibée ce qui se traduit par l'absence d'apparition de trouble ou culot bactérien.

Les croissances minimales inhibitrices (CMI) sont réalisées sur des souches bactériennes Gram positif selon les recommandations du « Clinical Laboratory Standards Institute » (Clinical-Laboratory-Standards-Institute, 6th ed. Approved standard M100-S17. CLSI, Wayne, PA, 2007).

### 3.1.5 Préparation de l'inoculum :

Les cultures étudiées, fraîchement isolées (après incubation sur une gélose de sang à 37°C pendant 18h), sont reprises dans de l'eau stérile (10 mL) jusqu'à obtenir une suspension à 0,5 Mac Farland (Mc) soit à 10⁸ UFC (bactérie)/cm³. La suspension bactérienne a ensuite été diluée afin d'obtenir une concentration finale de 10⁶ UFC/cm³.

### 3.2 Résultats

### 3.2.1 Résultats pour les souches du Genre des Staphylococcus

D'après l'observation des microplaques à 96 puits, tous les dérivés acétal ou éther de monosaccharides sont actifs contre les souches de *staphylocoques* testées (8 < CMI < 64 mg/L) à l'exception de l'éther de galactose (C12-Eth-α-MeGalac) et de l'acétal α du glucose (C12-Ac-α-MeGlu) (CMI >256 mg/L).

Lors de l'analyse des résultats (tableau 6), on notera que tous ces dérivés comprennent bien une chaine carbonée en C12. Or, seule une partie d'entre eux sont actifs contre les souches de staphylocoques testées (8 < CMI < 64 mg/L). En outre, lorsque l'on compare les molécules 1a et 1b, on constate alors que ces molécules ne se distinguent que par leur anomérie, seule l'une d'entre elle à une activité antibactérienne efficace.

**Tableau 6. Résultats antimicrobiens pour les dérivés d'éthers et acétals de glycopyranoside de méthyle ainsi que le sorbitane sur différentes souches de Staphylocoques S Aureus : Concentration Minimale Inhibitrice (CMI) en mg/L.**

| | *Staphylococcus* | | | | | | | | | | |
|---|---|---|---|---|---|---|---|---|---|---|---|
| | ATCC25923 | ATCC29213 | USA300 | MU3 | HT 2004-0012 | LY 199-0053 | HT 2002-0417 | HT 2006-1004 | ST 2015 0188 | ST 2014 1288 | ST 2015 0989 |
| C12-Ac-α-MeGlu | 256 | 256 | 256 | 256 | 256 | 256 | 256 | 256 | / | / | / |
| 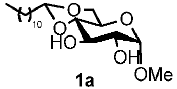 | | | | | | | | | | | |
| C12-Ac-β-MeGlu | 64 | 64 | 64 | 64 | | 64 | 128 | 64 | 64 | 64 | 64 |
| 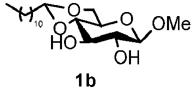 | | | | | | | | | | | |
| C12-Eth-α-MeGlu | 16 | 32 | 32 | 32 | 32 | 16 | 16 | 32 | 32 | 32 | 32 |
| 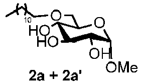 | | | | | | | | | | | |
| C12-Eth-α-MeMan | 32 | 32 | 32 | 64 | 32 | 32 | 32 | 64 | 64 | 32 | 64 |
| 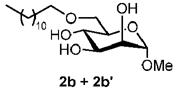 | | | | | | | | | | | |
| C12-Eth-α-MeGalac | 124 | 256 | 256 | 256 | 128 | 246 | 256 | 256 | 256 | 256 | 256 |
| 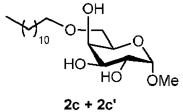 | | | | | | | | | | | |
| C12-Eth-α-MeRham | 32 | 16 | 32 | 64 | 16 | 32 | 32 | 32 | 64/32 | 32 | 64 |
| 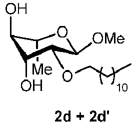 | | | | | | | | | | | |
| C12-Eth-Sorb | 32 | 32 | 32 | 64 | 32 | 32 | 32 | 32 | 64 | 64 | 256 |
| 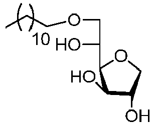 | | | | | | | | | | | |

De même, si l'on compare les molécules 1a et 2a+2a' qui se distinguent par la liaison éther ou acétal seule l'une de ces molécules a une activité antibactérienne efficace. Enfin, la nature du sucre est également susceptible de changer l'activité antibactérienne de la molécule. Ainsi, on note que les C12-Eth-α-MeGlu, C12-Eth-α-MeRham et C12-Eth-α-MeMan ont une activité antibactérienne comparativement auC12-Eth-α-MeGalac. Ces informations indiquent clairement que l'activité bactérienne d'une molécule est fonction de façon combinée de la nature du sucre, de la configuration du carbone anomérique et de la nature du lien avec la chaîne alkyle.

### Résultats pour les souches du Genre des Enterococcus

**Tableau 7. Résultats antimicrobiens pour les dérivés d'ethers de sucres et les acétals de sucres et de sorbitant sur différentes souches d'entérocoques. Concentration Minimale Inhibitrice (CMI) en mg/L**

| | **Enterococcus** | | | | | |
|---|---|---|---|---|---|---|
| | **ATCC29212** | **Van A** | **CIP 103510** | **1401** | **9901** | **1801** |
| C12-Ac-α-MeGlu | 256 | 256 | 256 | / | / | / |
| 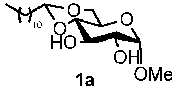 | | | | | | |
| C12-Ac-β-MeGlu | 64 | 32 | 32 | 16 | 32 | 8 |
| 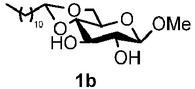 | | | | | | |
| C12-Eth-α-MeGlu | 16 | 16 | 16 | 8 | 16 | 8 |
| 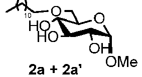 | | | | | | |
| C12-Eth-α-MeMan | 16 | 16 | 32 | 16 | 32 | 16 |
| 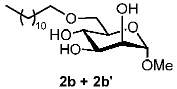 | | | | | | |
| C12-Eth-α-MeGalac | 64 | 124 | 256 | 32 | 64 | 8 |
| 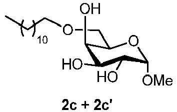 | | | | | | |
| C12-Eth-α-MeRham | 16 | 16 | 16 | 8 | 16 | 16 |
| 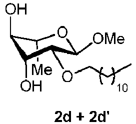 | | | | | | |
| C12-Eth-Sorb | 8 | 16 | 16 | 8 | 16 | 8 |
| 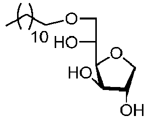 | | | | | | |

Une bonne activité antibactérienne est observée pour toutes les souches d'enterocoques 32 < CMI < 8 mg/L pour toutes les molécules testées à l'exception de C12-Ac-α-MeGlu et C12-Eth-α-MeGalac.

### Résultats pour les souches du genre des Listeria

**Tableau 8. Résultats antimicrobiens pour les dérivés d'ethers de sucres et les acétals de sucres et de sorbitant sur différentes souches de Listeria concentration Minimale Inhibitrice (CMI) en mg/L.**

| | **Listeria** | | | |
|---|---|---|---|---|
| | CIP 103575 | LM1 | LM2 | LM3 |
| C12-Ac-α-MeGlu | 64 | / | / | / |
| 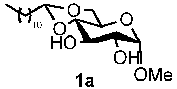 | | | | |
| C12-Ac-β-MeGlu | 16 | 16 | 16 | 64 |
| 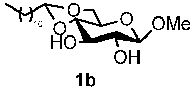 | | | | |
| C12-Eth-α-MeGlu | 8 | 8 | 8 | 8 |
| 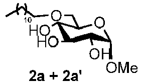 | | | | |
| C12-Eth-α-MeMan | 32 | 8 | 16 | 16 |
| 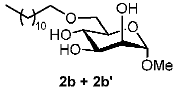 | | | | |
| C12-Eth-α-MeGalac | 64 | 64 | 64 | 64 |
| 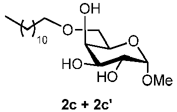 | | | | |
| C12-Eth-α-MeRham | 32 | 32 | 32 | 32 |
| 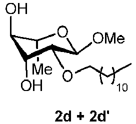 | | | | |
| C12-Eth-Sorb | 32 | 16 | 32 | 32 |
| 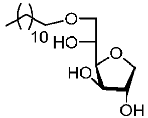 | | | | |

A l'exception des composés C12-Ac-α-MeGlu et C12-Eth-α-MeGalac, on notera qu'une bonne activité antibactérienne est observée sur toutes les souches de Listeria 64 < CMI < 8 mg/L pour toutes les molécules testées.

## Revendications

1. Composition **caractérisée en ce qu'**elle comprend un acétal d'alkyle ou un éther d'alkyle de désoxyhexose dans lequel le groupe alkyle comprend entre 11 à 18 atomes de carbone, un sel pharmaceutiquement acceptable, un isomère ou un mélange d'isomères de celui-ci, les isomères étant choisis parmi les régioisomères et/ou les diastéréoisomères, ledit radical acétal alkyle ou éther alkyle étant en position 2-*O*-, 3-*O*-, et/ou 4-*O*-.

2. Composition selon la revendication **1**, dans laquelle le désoxyhexose est le rhamnose ou le fucose.

3. Composition selon la revendication **1** ou **2**, dans laquelle le désoxyhexose est glycosylé et/ou hydrogéné et/ou déshydraté.

4. Composition selon l'une quelconque des revendications **1** à **3**, dans laquelle le désoxyhexose est glycosylé, préférentiellement le désoxy glycosylé étant le rhamnopyranoside de méthyle.

5. Composition selon l'une quelconque des revendications **1** à **4**, dans laquelle le groupe alkyle comprend 11 à 13 atomes de carbone.

6. Composition selon l'une quelconque des revendications **1** à **5**, dans laquelle l'acétal d'alkyle ou l'éther d'alkyle de désoxyhexose est un monoéther ou monoacétal d'alkyle de désoxyhexose préférentiellement, ledit radical mono acétal alkyle est en position 2,3-*O*- ou 3,4-*O*- ou ledit radical monoéther alkyle est en position 2-*O-*, 3-*O*- ou 4-*O*-.

7. Composition selon l'une quelconque des revendications **1** à **6** pour une utilisation comme agent bactéricide ou bactériostatique vis-à-vis des bactéries à Gram positif.

8. Composition selon la revendication **7**, **caractérisée en ce que** les bactéries à Gram positif sont des bactéries de l'embranchement des Firmicutes, typiquement de la classe des *Bacilli* notamment choisies parmi les bactéries de l'ordre des *Lactobacillales* ou des *Bacillales.*

9. Composition selon l'une ou l'autre des revendications **7** et **8**, **caractérisée en ce que** les bactéries à Gram positif sont des bactéries de l'ordre des Bacillales choisies parmi la famille des *Alicyclobacillaceae, Bacillaceae, Caryophanaceae, Listeriaceae, Paenibacillaceae, Pasteuriaceae, Planococcaceae, Sporolactobacillaceae, Staphylococcaceae, Thermoactinomycetacea* et *Turicibacteraceae* et/ou les bactéries à Gram positif sont des bactéries de l'ordre des *Lactobacillales* choisies parmi la famille des *Aerococcaceae, Carnobacteriaceae, Enterococcaceae, Lactobacillaceae, Leuconostocaceae* et *Streptococcaceae.*

10. Composition selon la revendication **9**, **caractérisée en ce que** les bactéries à Gram positif sont des bactéries de la famille des *Listeriaceae* telle qu'une bactérie du genre des *Brochothrix* ou des *Listeria* typiquement, choisies parmi *L. fleischmannii, L. grayi, L. innocua, L. ivanovii, L. marthii, L. monocytogenes, L. rocourtiae, L. seeligeri, L. weihenstephanensis* et *L. welshimeri* et/ou les bactéries à Gram positif sont des bactéries de la famille des *Staphylococcaceae* choisies parmi les bactéries du genre des *Staphylococcus, Gemella, Jeotgalicoccus, Macrococcus, Salinicoccus* et *Nosocomiicoccus* ; dans laquelle les bactéries du genre des *Staphylococcus* choisies parmi *S*. *arlettae, S. agnetis, S. aureus, S. auricularis, S. capitis, S. caprae, S. carnosus, S. caseolyticus, S. chromogenes, S. cohnii, S. condimenti, S. delphini, S. devriesei, S. epidermidis, S. equorum, S. felis, S. fleurettii, S. gallinarum, S. haemolyticus, S. hominis, S. hyicus, S. intermedius, S. kloosii, S. leei, S. lentus, S. lugdunensis, S. lutrae, S. massiliensis, S. microti, S. muscae, S. nepalensis, S. pasteuri, S. pettenkoferi, S. piscifermentans, S. pseudintermedius, S. pseudolugdunensis, S. pulvereri, S. rostri, S. saccharolyticus, S. saprophyticus, S. schleiferi, S. sciuri, S. simiae, S. simulans, S. stepanovicii, S. succinus, S. vitulinus, S. warneri* et *S*. *xylosus.*

11. Composition selon la revendication **9**, **caractérisée en ce que** les bactéries à Gram positif sont des bactéries de la famille des *Enterococcaceae* choisies parmi les bactéries du genre des *Bavariicoccus, Catellicoccus, Enterococcus, Melissococcus, Pilibacter, Tetragenococcus, Vagococcus* ; dans laquelle les bactéries du genre des *Enterococcus* choisies parmi *E. malodoratus, E. avium, E. durans, E. faecalis, E. faecium, E. gallinarum, E. hirae, E. solitarius,* préférentiellement, *E. avium, E. durans, E. faecalis* et *E. faecium.*

12. Composition selon l'une quelconque des revendications **1** à **6**, **caractérisée en ce qu'**elle est incorporée dans une composition alimentaire, cosmétique, pharmaceutique, phytosanitaire, vétérinaire ou de traitement de surface.

13. Composition selon l'une quelconque des revendications **1** à **6**, pour son utilisation comme produit d'hygiène ou dermatologique à usage externe et/ou pour son utilisation dans le traitement ou la prévention des infections bactériennes par des bactéries à Gram positif.

14. Composition selon la revendication **13**, dans laquelle, l'infection par des bactéries à Gram positif est une infection de la peau ou des muqueuses, préférentiellement une infection choisie parmi une folliculite, un abcès, un panaris, un furoncle, un impétigo, une infection interdigitale, un anthrax, une cellulite, une surinfection de plaies, un otitis sinusitis, une hydradénite, une mastite infectieuse, une infection post-traumatique de la peau et une infection de la peau brûlée.

15. Méthode de désinfection ou de prévention de la colonisation bactérienne par des bactéries à Gram positif d'un substrat comprenant la mise en contact du substrat avec une composition selon l'une quelconque des revendications **1** à **6** ; ledit substrat étant choisi parmi :
des aliments d'origine végétale ou animale ou une composition alimentaire comprenant de tels aliments ou un extrait de ces aliments, de préférence des extraits des céréales, des fruits, des légumes, de la viande, du poisson, des abats ;
des éléments sélectionnés parmi des métaux, des plastiques, du verre, du béton, de la pierre ;
des ustensiles, de préférence des outils ou des appareils utilisés dans l'industrie alimentaire ou hospitalière, de manière encore plus préférentielle, des outils de chirurgie ou des prothèses ; ou
des barres de maintien ou les sièges des transports en commun.

## Patentansprüche

1. Zusammensetzung, **dadurch gekennzeichnet, dass** sie ein Alkylacetal oder ein Desoxyhexosealkyether umfasst, in welchem die Alkylgruppe zwischen 11 und 18 Kohlenstoffatome, ein pharmazeutisch unbedenkliches Salz, ein Isomer oder eine Mischung von Isomeren davon umfasst, wobei die Isomere ausgewählt sind aus Regioisomeren und/oder Diastereomeren, wobei das Acetalalkylradikal oder Etheralkyl sich an der Position 2-*O*-, 3-*O*-, und/oder 4-*O*- befindet.

2. Zusammensetzung nach Anspruch **1**, in welcher die Desoxyhexose Rhamnose oder Fructose ist.

3. Zusammensetzung nach Anspruch **1** oder **2,** in welcher die Desoxyhexose glykolosiert und/oder hydrogenisiert und/oder dehydriert ist.

4. Zusammensetzung nach einem der Ansprüche **1** bis **3,** in welcher die Desoxyhexose glykolosiert ist, wobei das glykolisierte Desoxy bevorzugt Methylrhamnopyranosid ist.

5. Zusammensetzung nach einem der Ansprüche **1** bis **4,** in welcher die Alkylgruppe 11 bis 13 Kohlenstoffatome umfasst.

6. Zusammensetzung nach einem der Ansprüche **1** bis **5,** in welcher das Alkylacetal oder Desoxyhexosealkylether bevorzugt ein oder Desoxyhexosealkylonoether oder -monoacetal ist, wobei sich das Monoacetalalkylradikal an der Position 2,3-*O*- oder 3,4-*O*- befindet oder sich das Monoetheralkylradikal an der Position 2-*O*-, 3-*O*- oder 4-*O*- befindet.

7. Zusammensetzung nach einem der Ansprüche **1** bis **6** für eine Verwendung als bakterizides oder bakterostatisches Mittel gegenüber Gram-positiven Bakterien.

8. Zusammensetzung nach Anspruch **7, dadurch gekennzeichnet, dass** die Gram-positiven Bakterien Firmicutes-Verzweigungs-Bakterien sind, typischerweise aus der Klasse der *Bacilli,* insbesondere ausgewählt aus Bakterien der Ordnung der *Lactohacillales* oder der *Bacillales.*

9. Zusammensetzung nach einem der Ansprüche **7** oder **8, dadurch gekennzeichnet, dass** die Gram-positiven Bakterien Bakterien von der Ordnung der Bacillales sind, ausgewählt aus der Familie der *Alicyclohacillaceae, Bacillaceae, Caryphanaceae, Listeriaceae, Paenibacillaceae, Pasteuriaceae, Planococcaceae, Sporolactobacillaceae, Staphylococcacene, Thermoactinomycetacea* und *Turicibacteraceae* und/oder die Gram-positiven Bakterien aus der Ordnung der *Lactobacillales* sind, ausgewählt aus der Familie der *Aerococcaceae, Carnobacteriaceae, Enterococcaceae, Lactobacillaceae, Leuconostocaceae* und *Streptococcaceae.*

10. Zusammensetzung nach Anspruch **9, dadurch gekennzeichnet, dass** die Gram-positiven Bakterien typischerweise Bakterien aus der Familie der *Listeriaceae* sind wie ein Bakterium der Gattung der *Brochothrix* oder der *Listeria,* ausgewählt aus *L. fleischmannii, L. grayi, L. innocua, L. ivanovii, L. marthii, L. monocytogenes, L. rocourtiae, L. seeligeri, L. weihenstephanensis* und *L. welshimeri* sind und/oder die Gram-positiven Bakterien Bakterien aus der Familie der *Staphylococcaceae* sind, ausgewählt aus den Bakterien der Gattung der *Staphylococcus, Gemella, Jeotgalicoccus, Macrococcus, Salinicoccus* und *Nosocomiicoccus;* in welcher die Bakterien der Gattung der *Staphylococcus* ausgewählt sind aus *S. arlettae, S. agnetis, S. aureus, S. auricularis, S. capitis, S. caprae, S. carnosus, S. caseolyticus, S. chromogenes, S. cohnii, S. condimenti, S. delphini, S. devriesei, S. epidermidis, S. equorum, S. felis, S. fleurettii, S. gallinaruin, S. haemolyticus, S. hominis, S. hyicus, S. intermedius, S. kloosii, S. leei, S. lentus, S. lugdunesis, S. lutrae, S. massilensis, S. microti, S. muscae, S. nepalensis, S. pasteuri, S. pettenkoferi, S. piscifermantans, S. pseudintermedius, S. pseudolugdunensis, S. pulvereri, S. rostri, 3. saccharolyticus, S. saprophyticus, S. schleiferi, S. sciuri, S. siminae, S. simulans, S. stepanovicii, S. succinus, S. vitulinus, S. warneri* und *S. xylosus.*

11. Zusammensetzung nach Anspruch **9, dadurch gekennzeichnet, dass** die Gram-positiven Bakterien Bakterien aus der Familie der *Enterococcaceae* sind, ausgewählt aus den Bakterien der Gattung der *Bavariicoccus, Catellicoccus, Enterococcus, Melissococcus, Pilibacter, Tetragenococcus, Vagococcus;* in welcher die Bakterien der Gattung des *Enterococcus* ausgewählt sind aus *E. malodoratus, E. avium, E. durans, E. faecalis, E. faecium, E. gallinarum, E. hirae, E. solitarius,* bevorzugt, *E. avium, E. durans, E. faecalis* und *E. faecium.*

12. Zusammensetzung nach einem der Ansprüche **1** bis **6, dadurch gekennzeichnet, dass** sie in eine alimentäre, kosmetische, pharmazeutische, phytosanitäre, Veterinäre oder Oberflächenbehandlungs-Zusammensetzung implementiert wird.

13. Zusammensetzung nach einem der Ansprüche **1** bis **6** für die Verwendung als Hygiene- oder dermatologisches Produkt zur externen Anwendung und/oder zur Verwendung bei der Behandlung oder der Vorbeugung von bakteriellen Infektionen durch Gram-positive Bakterien.

14. Zusammensetzung nach Anspruch **13**, in welcher die Infektion durch Gram-positive Bakterien eine Infektion der Haut oder der Mukosen ist, bevorzugt eine Infektion ausgewählt aus einer Follikulitis, einem Abszess, einer Paronychie, einem Furunkel, einem Impetigo, einer interdigitalen Infektion, einem Anthrax, einer Cellulitis, einer Wund-Superinfektion, einer Otitis Sinusitis, einer Acne inversa, einer infektiösen Mastitis, einer posttraumatischen Hautinfektion und einer Entzündung von verbrannter Haut.

15. Verfahren zur Desinfektion oder Prävention von bakterieller Besiedelung durch Gram-positive Bakterien durch ein Substrat, umfassend das Inkontaktbringen des Substrats mit einer Zusammensetzung nach einem der Ansprüche **1** bis **6,** wobei das Substrat ausgewählt ist aus:
Lebensmitteln pflanzlichen oder tierischen Ursprungs oder einer solche Lebensmittel umfassenden alimentären Zusammensetzung oder einem Extrakt dieser Lebensmittel, bevorzugt Extrakte von Getreide, Früchten, Gemüse, Fleisch, Fisch, Schlachtabfällen;
Elementen ausgewählt aus Metallen, Kunststoffen, Glas, Beton, Stein;
Utensilien, bevorzugt in der Lebensmittel- oder Gastgewerbeindustrie verwendete Geräte oder Apparate, noch bevorzugter Chirurgiewerkzeuge oder Prothesen; oder Haltestangen oder Sitze von öffentlichen Verkehrsmitteln.

## Claims

1. A composition, **characterized in that** it comprises a deoxyhexose alkyl ether or alkyl acetal wherein the alkyl group comprises between 11 and 18 carbon atoms, a pharmaceutically acceptable salt, an isomer or a mixture of isomers thereof, the isomers being selected from regioisomers and/or diastereoisomers; said alkyl ether or alkyl acetal radical being on the 2-*O*-, 3-*O*-, and/or 4-*O*-position.

2. The composition as claimed in claim **1,** wherein the deoxyhexose is rhamnose or fucose.

3. The composition as claimed in either one of claims **1** or **2,** wherein the deoxyhexose is glycosylated and/or hydrogenated and/or dehydrated.

4. The composition as claimed in any one of claims **1** to **3,** wherein the deoxyhexose is glycosylated, preferably the glycosylated deoxy being methyl rhamnopyranoside.

5. The composition as claimed in any one of claims **1** to **4,** wherein the alkyl group comprises 11 to 13 carbon atoms.

6. The composition as claimed in any one of claims **1** to **5, characterized in that** the deoxyhexose alkyl ether or alkyl acetal is a deoxyhexose alkyl monoacetal or monoether; preferably, said alkyl monoacetal radical is in the 2,3-*O*- or 3,4-*O*-position, or said alkyl monoether radical is in the 2-*O*-, 3-*O*- or 4-*O*-position.

7. The composition as claimed in any one of claims **1** to **6,** for use as bactericidal or bacteriostatic agent against Gram-positive bacteria.

8. The composition as claimed in claim 7, **characterized in that** the Gram-positive bacteria are bacteria from the phylum Firmicutes, typically of the *Bacilli* class, namely selected from bacteria of the *Lactobacillales* or *Bacillales* order.

9. The composition as claimed in either one of claims **7** or **8, characterized in that** the Gram-positive bacteria are bacteria of the order Bacillales selected from the family *Alicyclobacillaceae, Bacillaceae, Caryophanaceae, Listeriaceae, Paenibacillaceae, Pasteuriaceae, Planococcaceae, Sporolactobacillaceae, Staphylococcaceae, Thermoactinomycetacea* and *Turicibacteraceae,* and/or the Gram-positive bacteria are bacteria of the order *Lactobacillales* selected from the families of *Aerococcaceae, Carnobacteriaceae, Enterococcaceae, Lactobacillaceae, Leuconostocaceae* and *Streptococcaceae.*

10. The composition as claimed in claim **9, characterized in that** the Gram-positive bacteria are bacteria of the *Listeriaceae* family , such as a bacterium of the *Brochothrix* or *Listeria* genus, typically selected from *L. fleischmannii, L. grayi, L. innocua, L. ivanovii, L. marthii, L. monocytogenes, L. rocourtiae, L. seeligeri, L. weihenstephanensis* and *L. welshimeri* and/or the Gram-positive bacteria are bacteria of the *Staphylococcaceae* family selected from bacteria of the genera *Staphylococcus, Gemella, Jeotgalicoccus, Macrococcus, Salinicoccus* and *Nosocomiicoccus;* wherein the bacteria of the genus *Staphylococcus* are selected from *S. arlettae, S. agnetis, S. aureus, S. auricularis, S. capitis, S. caprae, S.carnosus, S. caseolyticus, S. chromogenes, S. cohnii, S. condimenti, Sdelphini, S. devriesei, S. epidermidis, S. equorum, S. fells, S. fleurettii, S.gallinarum, S. haemolyticus, S. hominis, S. hyicus, S. intermedius, S. kloosii, S. leei, S. lentus, S. lugdunensis, S. lutrae, S. massiliensis, S. microti, Smuscae, S. nepalensis, S. pasteuri, S. pettenkoferi, S. piscifermentans, S. pseudintermedius, S. pseudolugdunensis, S. pulvereri, S. rostri, S. saccharolyticus, S. saprophyticus, S. schleiferi, S. sciuri, S. simiae, S. simulans, S. stepanovicii, S. succinus, S. vitulinus, S. warneri* and *S. xylosus.*

11. The composition as claimed in claim **9, characterized in that** the Gram-positive bacteria are bacteria of the *Enterococcaceae* family, selected from the bacteria of the genera *Bavariicoccus, Catellicoccus, Enterococcus, Melissococcus, Pilibacter, Tetragenococcus,* or *Vagococcus;* wherein the bacteria of the genus *Enterococcus* are selected from *E. malodoratus, E. avium, E. durans, E. faecalis, E. faecium, E. gallinarum, E. hirae, E. solitarius,* preferably *E. avium, E. durans, E. faecalis* and *E. faecium.*

12. The composition as claimed in any one of claims **1** to **6, characterized in that** it is incorporated into a food, cosmetic, pharmaceutical, phytosanitary, veterinary or surface treatment composition.

13. The composition as claimed in any one of claims **1** to **6,** for use thereof as a hygiene or dermatological product for external use and/or for use in treating or preventing bacterial infections by Gram-positive bacteria.

14. The composition as claimed in claim **13,** wherein the infection by Gram-positive bacteria is an infection of the skin or the mucous membranes, preferably an infection selected from folliculitis, an abscess, paronychia, a boil, impetigo, an interdigital infection, anthrax, cellulitis, a secondary wound infection, otitis sinusitis, hidradenitis, infectious mastitis, a post-traumatic skin infection or an infection of burnt skin.

15. A method for disinfecting or for preventing bacterial colonization by Gram-positive bacteria of a substrate comprising bringing the substrate into contact with a composition as claimed in any one of claims **1** to **6,** said substrate being selected from:
foods of plant or animal origin or a food composition comprising such foods or an extract of these foods, preferably extracts of cereals, fruits, vegetables, meat, fish, offal;
elements selected from metals, plastics, glass, concrete, stone;
utensils, preferably tools or devices used in the food or hospital industry, more preferably, surgical tools or prostheses; or
hand rails or public transport seats.
